# FASCICULE DE BREVET EUROPEEN

(11) **EP 1 887 890 B1**
(45) Date de publication et mention de la délivrance du brevet: **02.07.2014**
(21) Numéro de dépôt: 06794430.6
(22) Date de dépôt: 15.05.2006
(51) Int. Cl.: A61K 8/37, A61Q 19/00, A61Q 19/08, A61K 8/92, A61K 8/02, A23L 1/30

(54) **TRAITEMENT DE LA SECHERESSE KERATINIQUE PAR DES GLYCERIDES**
BEHANDLUNG VON TROCKENHEIT DES KERATINGEWEBES ÜBER GLYCERIDE
TREATING KERATINOUS DRYNESS USING GLYCERIDES

(30) Priorité: 16.05.2005 FR 0551264; 28.12.2005 US 754214 P
(43) Date de publication de la demande: 20.02.2008
(73) Titulaire: Laboratoires Inneov, 92602 Asniéres Cedex (FR)
(72) Inventeur: MANISSIER, Patricia, F-92300 Levallois-Perret (FR); MONTASTIER, Christiane, F-75016 Paris (FR); PICCIRILLI, Antoine, F-86000 POITIERS (FR)
(74) Mandataire: Le Coupanec, Pascale A.M.P.
(86) Numéro de dépôt international: PCT/FR2006/050445
(87) Numéro de publication internationale: WO 2007/000529

(56) Documents cités:
- EP-A- 1 023 843
- EP-A- 1 214 930
- WO-A-2006/002050
- CA-A1- 1 253 807
- DE-A1- 19 857 491
- US-A- 5 223 285
- US-A1- 2004 151 757
- US-A1- 2005 143 459
- US-B1- 6 194 379
- US-B1- 6 365 175
- DATABASE WPI Section Ch, Week 200532 Derwent Publications Ltd., London, GB; Class B05, AN 2005-310723 XP002370666 & JP 2005 104928 A (BUNSHI SEIRI KAGAKU KENKYUSHO KK) 21 avril 2005 (2005-04-21)

## Description

La présente description concerne à titre principal l'utilisation de glycérides à chaîne moyenne pour la préparation d'une composition pour l'absorption orale et/ou parentérale, destinée à prévenir et/ou traiter la sécheresse des matières kératiniques, en particulier la sécheresse cutanée et notamment à traiter les désordres liés à une peau sèche et/ou hypo-séborrhéique.

On rappelle que la peau est constituée de trois couches superposées, de la surface vers la profondeur du corps : l'épiderme, le derme et l'hypoderme et comporte en outre des structures annexes telles que notamment les glandes sébacées.

La peau exerce essentiellement une fonction de barrière vis-à-vis du milieu extérieur qui résulte d'une organisation complexe et de nombreux facteurs. Cette fonction repose en particulier sur la qualité de l'épiderme qui dépend notamment du taux d'hydrophobicité de surface du stratum corneum et de l'équilibre entre la prolifération et la différenciation des kératinocytes épidermique.

La rupture de l'équilibre cutané peut se manifester de différentes façons. Elle peut notamment conduire au déclenchement de processus inflammatoires, d'un dérèglement de la fonction sébacée, d'hyperkératinisation, ainsi qu'à une augmentation de la perte insensible en eau et plus généralement à une sécheresse cutanée. Ces événement affectent de façon négative le confort et/ou l'esthétique cutanée. Ils sont en outre susceptibles d'affecter l'état sanitaire de l'épiderme.

Ainsi, l'altération de la fonction barrière, va favoriser la pénétration anormale d'agents pathogènes dans la couche cornée et induire une libération accrue de substances pro-inflammaboires à l'origine de désordres inflammatoires cutanés qui provoquent des démangeaisons et tiraillements, deux symptômes caractéristiques d'une peau sèche.

Une première alternative de traitement des peaux sèches caractérisées par une déficience en lipides constitutifs de la barrière et/ou du film hydrolipidique, vise à administrer par voie topique des produits destinés à restaurer la barrière cutanée. Ces produits sont généralement des agents humidifiants, capables de fixer l'eau, des agents filmogènes destinés à retenir l'eau, des agents capables de reconstruire la barrière cutanée tels que les lipides exogènes constitutifs du ciment intercellulaire et du sébum tels que le squalène, les céramides, les acides gras ou encore des actifs à l'image de la vitamine C capables de stimuler la synthèse endogène des lipides épidermiques.

Toutefois ce mode d'administration implique le renouvellement fréquent des applications topiques, possède une efficacité limitée à la zone bénéficiant de l'application topique et peut en outre générer des effets cutanés secondaires indésirables.

Une autre alternative consiste à supplémenter, généralement par voie orale, en acides gras en particulier polyinsaturés dits AGPI des familles ω3 et ω6 les sujets affectés. Dans la peau, ces acides gras, vont subir, sous l'action d'enzymes de type élongase et desaturase, des biotransformations pour conduire à des polyènes supérieurs pro- ou anti-inflammatoires comme les acides eicosapentaoïque EPA, et docosahexaénoïque DHA, les prostaglandines, les leucotriènes. Il a ainsi été montré que chez l'enfant atopique, une supplémentation en huile de pépins de raisin, riche en acide stéaridonique et acide gamma linolénique, deux précurseurs de prostaglandines, permettait de corriger les désordres inflammatoires cutanés (F. Balli et al. Riv. Ital. Pediatr. (IJP) 1992 ; 18:639-643). De même, la consommation à raison de 2400 mg/jour d'huile de pépins de cassis, riches également en acides gamma linolénique et stéaridonique, s'est montrée active pour le traitement de la xérose ou de la peau sèche, en particulier chez le sujet âgé (Wade, The Nutrition & Dietary Consultant, Sept 1986, pages 4-17).

Comme précisé précédemment, la peau sèche peut être également la conséquence et/ou être associée à une insuffisance endogène de production de sébum par les glandes sébacées.

Conjointement à la sueur, le sébum constitue un hydratant naturel de l'épiderme et permet d'en accroître la souplesse et la résistance. Il est composé pour l'essentiel d'un mélange plus ou moins complexe de lipides. Classiquement, la glande sébacée produit du squalène, des triglycérides, des cires aliphatiques, des cires de cholestérol et, éventuellement, du cholestérol libre. C'est l'action des lipases bactériennes qui convertit une part variable des triglycérides formés en acides gras libres.

Classiquement, un taux de sébum inférieur à 100 µg/cm², mesuré au niveau de la zone T du visage, par la méthode décrite dans FR 2 368 708, peut être considéré comme caractéristique d'une peau sèche hypo-séborrhéique.

Un exemple de peau sèche hypo-séborrhéique, ou de peau le devenant est observé au cours du vieillissement cutané. Ainsi, il est très souvent constaté chez les sujets âgés et notamment de plus de 50 ans, la manifestation d'une xérose liée à une déficience en sébum. Par ailleurs, l'insuffisance de production de sébum peut être induite, par certains traitements pharmaceutiques comme ceux impliquant des corticoides.

L'utilisation, notamment par voie topique ou orale, des amides ou esters de sucre et d'acides gras et en particulier d'acide linolénique est également proposée dans le document WO 04/034958 pour le traitement de la sécheresse cutanée et notamment de la peau sèche hypo-séborrhéique.

La présente invention vise pour sa part à proposer l'utilisation d'autres composés dont l'efficacité a été constatée de manière inattendue pour la prévention et/ou le traitement d'une manière générale des désordres associés à la sécheresse des matières kératiniques.

Au sens de la présente invention, "matière kératinique" désigne la peau, le cuir chevelu, les muqueuses, les ongles, et les fibres kératiniques d'origine humaine ou animale.

Plus précisément, les inventeurs ont mis en évidence que des glycérides à chaîne moyenne, c'est-à-dire composés d'acides gras dont la chaîne hydrocarbonée comprend de 6 à 12 atomes de carbone, et en particulier les triglycérides à chaîne moyenne, s'avèrent efficaces pour le traitement des matières kératiniques sèches et/ou fragilisées. De manière inattendue, leur administration par voie orale permet de relancer la production de sébum.

Les glycérides à chaîne moyenne sont des composés dérivant de l'estérification du glycérol par une, deux ou trois molécules d'acides gras à chaîne moyenne.

Les triglycérides de chaîne moyenne, encore communément désignés par l'abréviation TCM sont composés de trois molécules d'acide(s) gras à chaîne moyenne estérifiées par une molécule de glycérol. Ce sont des produits sans odeur, sans goût, limpides et possédant une faible viscosité.

Les triglycérides à chaîne moyenne sont déjà connus en tant que sources rapides et directes d'approvisionnement énergétique, notamment en raison de leur profil métabolique unique. En effet, comparativement aux triglycérides d'acides gras à longue chaîne, les triglycérides à chaîne moyenne libèrent après ingestion des acides gras courts, nettement moins hydrophobes que les acides gras à longue chaîne et qui parviennent directement au foie via la veine porte sans être incorporés auparavant dans les transporteurs que sont les lipoprotéines.

Ainsi, ils ont été proposés dans EP 1 023 843 pour la formulation de compléments nutritionnels plus particulièrement dédiés aux personnes pratiquant une activité sportive.

Les TCM sont également déjà proposés à titre de source en matières grasses dans les produits nutritionnels médicaux (US 2004/0151757 ou US 5,223,285). Notamment, leur bonne digestibilité du fait de leur hydrolyse en partie par les lipases linguales et gastriques et leur rapide métabolisme font de leur utilisation un moyen efficace d'apport énergétique dans les situations de malabsorption (US 6,194,379). Ils sont également proposées pour des annulations nutritionnelles à teneur en calorie et/ou en graisse réduite dédiées plus particulièrement à des personnes souffrant de surcharge pondérale (US 2005/0143459).

Par ailleurs, ces composés ont également été proposés pour un usage dermatologique par voie topique (DE 198 57491, CA 1 253 807, EP 1 214 930 ou JP 2005-104928).

De manière inattendue, les inventeurs ont constaté que ces composés s'avéraient en outre particulièrement efficaces, lorsqu'ils étaient administrés par voie non topique, et en particulier par voie orale et/ou parentérale, pour traiter des désordres liés à des matières kératiniques sèches ou fragilisées, en particulier à la peau sèche, et plus particulièrement la peau sèche associée notamment à un taux de sébum bas.

En conséquence, la présente invention concerne l'utilisation de glycéride(s) à chaîne moyenne, en tant qu'actif pour la préparation d'une composition pour l'absorption orale et/ou parentérale et destinée à prévenir et/ou traiter les matières kératiniques sèches et/ou fragilisées.

La présente description concerne, selon un autre de ses aspects, l'utilisation de glycéride(s) à chaîne moyenne, en tant qu'actif pour la préparation d'une composition pour l'absorption orale et/ou parentérale et destinée à prévenir et/ou traiter les peaux sèches et/ou fragilisées.

On décrit aussi l'utilisation de glycéride(s) à chaîne moyenne, en tant qu'actif, pour la préparation d'une composition pour l'absorption orale et/ou parentérale et destinée à traiter et/ou à prévenir des désordres cutanés liés à un défaut d'excrétion et/ou de sécrétion de sébum.

La présente invention concerne l'utilisation non thérapeutique de glycéride(s) à chaîne moyenne, à titre d'actif, par voie orale, pour prévenir et/ou traiter les matières kératiniques sèches et/ou fragilisées, en particulier la peau.

La présente invention concerne l'utilisation de glycéride(s) à chaîne moyenne, en tant qu'actif, pour la préparation d'une composition thérapeutique pour l'absorption orale et/ou parentérale, et destinée à prévenir et/ou traiter les peaux sèches et/ou fragilisées.

On décrit également, selon un autre aspect l'utilisation de glycéride(s) à chaîne moyenne, en tant qu'actif pour la préparation d'une composition pour l'absorption orale et/ou parentérale et destinée à traiter les peaux sèches hypo-séborrhéiques.

On décrit encore, selon un autre aspect l'utilisation de glycéride(s) à chaîne moyenne, en tant qu'actif pour la préparation d'une composition pour l'absorption oraleet/ou parentérale et destinée à stimuler la sébogénèse.

La présente description concerne en outre l'utilisation de glycéride(s) à chaîne moyenne, en tant qu'actif, pour la préparation d'une composition pour l'absorption orale et/ou parentérale et destinée à prévenir et/ou traiter les démangeaisons et/ou tiraillements.

Selon encore un autre de ses aspects, la présente description concerne l'utilisation de glycéride(s) à chaîne moyenne, en tant qu'actif pour la préparation d'une composition pour l'absorption orale et/ou parentérale et destinée à prévenir et/ou traiter les fibres kératiniques sèches ou fragilisées et/ou les désordres foncftionnels de l'unité pilo-sébacée.

Selon encore un autre de ses aspects, la présente description concerne l'utilisation de glycéride(s) à chaîne moyenne, en tant qu'actif, pour la préparation d'une composition pour l'absorption orale et/ou parentérale et destinée à prévenir et/ou réduire les rides liées à une sécheresse cutanée.

Par "fibres kératiniques", on entend désigner les cheveux, les poils et les cils humains ou animals.

Les compositions selon l'invention peuvent se présenter sous la forme d'un complément alimentaire, d'une composition nutritionnelle ou sous la forme d'un aliment functionnel à visée de traitement des matières kératiniques et notamment de la peau, et destinés aux êtres humains ou aux animaux.

Elles peuvent également se présenter sous la forme d'une composition à caractère thérapeutique.

La présente description concerne également selon un autre de ses aspects, l'utilisation thérapeutique ou non de glycéride(s) à chaîne moyenne, à titre d'actif pour prévenir et/ou traiter les matières kératiniques sèches et/ou fragilisées, en particulier les peaux sèches et/ou fragilisées, et/ou pour prévenir et/ou traiter les démangeaisons et/ou tiraillements et/ou pour le traitement et/ou la prévention des désordres cutanés et/ou de l'unité pilo/sébacée liés à un défaut d'excrétion et/ou de sécrétion de sébum, et/ou pour traiter les peaux sèches hypo-séborrhéiques et/ou pour stimuler la sébogénèse, et/ou pour prévenir et/ou traiter les fibres kératiniques sèches ou fragilisées, et/ou pour prévenir et/ou réduire les rides liées à une sécheresse cutanée.

Plus particulièrement, une utilisation selon l'invention est effectuée par absorption par voie orale et/ou parentérale.

On décrit aussi une composition pour l'absorption orale et destinée à prévenir et/ou traiter les matières kératiniques sèches ou fragilisées, et notamment les peaux sèches et/ou fragilisées, caractérisée en ce qu'elle comprend à titre d'actif au moins un glycéride à chaîne moyenne et au moins un autre agent actif au niveau de la peau.

La présente invention a également pour objet une méthode ou procédé non thérapeutique pour prévenir et/ou de traiter des désordres des matières et/ou des fibres kératiniques, notamment tels que précités, et/ou stimuler la sébogénèse comprenant l'administration d'au moins une quantité efficace de glycéride(s) à chaîne moyenne.

Ainsi, la présente invention a en outre pour objet un procédé de traitement non thérapeutique pour prévenir et/ou traiter les matières kératiniques sèches et/ou fragilisées, et en particulier les peaux sèches et/ou fragilisées, et/ou pour traiter les peaux sèches hypo-séborrhéiques, et/ou pour prévenir et/ou traiter les démangeaisons et/ou tiraillements, et/ou pour stimuler la sébogénèse, et/ou pour le traitement et/ou la prévention des désordres cutanés et/ou de l'unité pilo/sébacée liés à un défaut d'excrétion et/ou de sécrétion de sébum, et/ou pour le traitement et/ou la prévention des fibres kératiniques sèches ou fragilisées, et/ou pour prévenir et/ou réduire les rides liées à une sécheresse cutanée comprenant l'administration d'au moins une quantité efficace de glycéride(s) à chaîne moyenne.

L'administration peut notamment être effectuée par voie orale ou parentérale et est réalisée plus particulièrement par voie orale.

### GLYCERIDES A CHAINE MOYENNE

Les glycérides couvrent d'une manière générale les monoglycérides, les diglycérides, les triglycérides et leurs mélanges.

Selon un mode de réalisation préféré de l'invention, il s'agit plus particulièrement de triglycérides à chaîne moyenne, le cas échéant en mélange avec des monoglycérides et/ou des diglycérides.

Les glycérides à chaîne moyenne mis en oeuvre dans le cadre de la présente invention se présentent d'une manière générale sous la forme de mélanges.

Ils sont composés, selon leur degré d'estérification, d'une ou plusieurs molécules d'acides gras à chaîne moyenne, c'est-à-dire d'acides gras dont le nombre d'atomes de carbone varient de 6 à 12, et en particulier de 8 à 10 par opposition à un acide gras à chaîne longue qui possède un nombre d'atomes de carbone supérieur à 14 et un acides gras à chaîne courte qui possède un nombre d'atomes de carbone compris entre 4 et 6.

Les acides gras à chaîne moyenne peuvent être constitués de chaînes linéaires ou ramifiées, saturées ou insaturées, pouvant comporter une ou plusieurs insaturations.

Plus particulièrement, les acides gras conformes à l'invention possèdent des chaînes linéaires et saturées.

Les glycérides à chaîne moyenne sont ainsi généralement composés d'acides gras à chaîne moyenne s'étendant de l'acide caproïque (C₆) à l'acide laurique (C₁₂).

Les glycérides considérés selon l'invention peuvent être composés d'un unique type d'acide gras, notamment en C₆, C₈, C₁₀ ou C₁₂ ou, dans le cas des diglycérides et triglycérides, dérivés de l'estérification du glycérol par des molécules d'acide gras de longueur de chaînes différentes.

Plus précisément, les glycérides à chaîne moyenne et en particulier les triglycérides à chaîne moyenne convenant à la présente invention sont composés de 40 à 90 %, en particulier de 45 à 75 %, et de préférence de 50 à 70 % en poids d'acides gras en C₈. Ils possèdent également de 15 à 60 %, et en particulier de 30 à 50 % d'acides gras en C₁₀.

Généralement ils peuvent se présenter sous la forme d'un mélange comprenant au maximum 2 % en poids d'acides gras en C₆.

De même, les mélanges de glycérides à chaîne moyenne conformes à la présente invention possèdent généralement moins de 3 % et en particulier au plus 1 % en poids d'acides gras en C₁₂.

L'exemple 1 ci-après propose une composition d'un mélange de triglycérides à chaîne moyenne conforme à l'invention.

D'une manière générale, les triglycérides à chaîne moyenne mis en oeuvre dans la présente invention sont utilisés sous la forme de mélanges de plusieurs triglycérides à chaîne moyenne et dont les proportions respectives peuvent varier selon les mélanges.

De tels mélanges sont généralement obtenus à l'issu de leur procédé de fabrication.

Généralement, les triglycérides à chaîne moyenne sont préparés par estérification du glycérol avec des acides gras à chaîne moyenne issus des huiles à teneur élevée en acide laurique. Les huiles de noix de coco et de palmiste qui contiennent respectivement environ 13 % et environ 8 % en acides gras en C₈/C₁₀ ou encore les huiles de cuphea qui peuvent contenir en fonction de leur variété, jusqu'à 87 % de C₈ (pour le *cuphea painteri*) et 81 % de C₁₀ (pour le *cuphea carthagenensis*) sont parmi les sources naturelles les plus riches et les plus abondantes en acides gras à chaîne moyenne. Classiquement, ces huiles sont hydrolysées pour libérer leurs acides gras du glycérol, puis ces acides gras sont ensuite séparés par distillation fractionnée. La fraction de tête des acides gras contient les acides gras à chaîne moyenne. Des fractions d'acides en C₈ et C₁₀ contenant seulement de petites quantités en acides en C₆ et C₁₂ peuvent ainsi être isolées. Une réaction d'estérification entre le glycérol et les acides gras à chaîne moyenne, ainsi isolés, permet d'obtenir des mélanges de triglycérides à chaîne moyenne. Différentes proportions en chacun de ces acides peuvent être obtenues pour conduire à des produits différents. Des produits contenant de manière prédominante soit la forme en C₈ soit la forme en C₁₀, de même que des mélanges de ces deux formes, sont commercialement disponibles.

Ces triglycérides à chaîne moyenne sont encore couramment désignés sous le terme "captrin" tri(caprylate/capra) de glycéryle ou triglycéride caprique/caprylique.

A titre illustratif des produits à base de triglycérides convenant à l'invention, on peut particulièrement citer ceux commercialisés sous la dénomination commerciale NEOBEE® M-5 de la STEPAN COMPANY OF MAYWOOD, la dénomination CAPTEX® de ABITEC COMPANY, ou encore la dénomination Delios^{®} V de la société COGNIS.

Les glycérides peuvent également être mis en oeuvre sous une forme non isolée. Auquel cas, ils peuvent être présents dans les compositions selon l'invention sous la forme d'au moins une huile végétale ou d'un mélange d'huiles végétales les contenant et notamment riche en acide gras à chaîne moyenne en C₈ à C₁₀.

Par exemple, il peut s'agir d'huiles de coco, de cuphea, de palmiste et de babassu.

Selon ce mode de réalisation, la quantité en huile est ajustée pour procurer une quantité en glycérides et plus particulièrement en TCM suffisante pour obtenir l'effet attendu.

Par ailleurs, les glycérides peuvent être utilisés sous une forme pure ou associée avec d'autre(s) composé(s) au sein d'une composition, pour les utilisations considérées selon l'invention.

Au sens de l'invention, on entend désigner par l'expression « quantité efficace », la quantité minimum nécessaire à l'observation de l'effet attendu.

Une telle quantité peut aisément être déterminée par l'homme de l'art.

Au sein d'une composition, les glycérides et plus particulièrement les TCM peuvent représenter de 0,1 à 100 % en poids, en particulier de 15 à 80 % et notamment de 30 à 70 % en poids par rapport au poids total de la composition.

Quelque soit la variante considérée, les glycérides et plus particulièrement les TCM sont généralement administrés à des doses journalières pouvant aller de 0,5 mg à 100 g/j, en particulier de 1 mg à 10 g/j, notamment de 5 mg à 2,5 g/j.

Par opposition à la voie d'administration plus conventionnelle qu'est la voie topique, la voie orale a pour avantage d'être d'une part facile et rapide d'utilisation et d'autre part efficace de façon globale sur l'ensemble des matières kératiniques.

Selon un mode de réalisation, une composition conforme comprend moins de 2 % en poids d'acide pétrosélinique par rapport au poids total de la composition, voire est dépourvue d'acide pétrosélinique

Selon un mode de réalisation, une composition peut comprendre moins de 2 % en poids d'huile de vaseline par rapport au poids total de la composition, voire peut être dépourvue d'huile de vaseline.

Selon un mode de réalisation, une composition peut être dépourvue d'agent actif de type de filtre UV.

Selon un mode de réalisation, une composition peut être dépourvue d'un coenzyme Q.

Selon un mode de réalisation, une composition peut être dépourvue d'un agent conservateur de la classe des paraben (par exemple méthylparaben ou propylparaben).

Selon un mode de réalisation, une composition peut être dépourvue de chrome ou d'un sel de chrome (chlorure de chrome (III), 6H₂O)*.*

Selon un mode de réalisation, une composition peut être dépourvue d'hydrolysat de caseinate de sodium.

Lorsque les matières kératiniques considérées selon l'invention sont des fibres kératiniques humaines ou animales, à l'image des cheveux, poils et/ou cils, les glycérides à chaînes moyennes s'avèrent particulièrement avantageux pour prévenir et/ou traiter la manifestation des signes de fragilité, comme par exemple la sécheresse qui se traduit, généralement, par un aspect cassant de la fibre.

Les glycérides à chaînes moyenne permettent de conférer un aspect lustré aux fibres kératiniques, en particulier à la chevelure humaine et au pelage animal.

Dans le cas particulier de la peau sèche, les glycérides à chaîne moyenne s'avèrent particulièrement efficaces pour prévenir et/ou traiter les peaux sèches ou les peaux fragilisées et notamment pour restaurer physiologiquement un état d'hydratation convenable au *stratum corneum.*

Au sens de l'invention, une peau fragilisée est une peau affectée par un déficit de la fonction barrière et notamment caractérisée, ou susceptible de l'étre, par une déficience en lipides constitutifs de la barrière et/ou du film hydrolipidique. Toutefois, une peau fragilisée ne manifeste pas, ou du moins à échelle égale, les symptômes caractéristiques de la peau sèche à savoir démangeaisons et tiraillements.

Par ailleurs, une peau fragilisée est différente d'une part d'une peau allergique et d'autre part d'une peau dite sensible. En effet, sa réactivité ne relève pas d'un processus immunologique à l'image des peaux allergiques, ni d'un processus neurogéne à l'image des peaux dites sensibles.

Ainsi, sont visées par la présente invention des matières kératiniques sèches et/ou fragilisées distinctes des peaux allergiques ou sensibles, sèches ou non.

La sécheresse susceptible d'être traitée selon l'invention peut être une sécheresse acquise et passagère, c'est-à-dire une sécheresse liée à une déshydratation de la peau par exemple par le froid, la chaleur, les détergents, l'eau calcaire et/ou les produits chimiques tels que les solvants.

Il peut également s'agir d'une sécheresse acquise et permanente comme par exemple celle due à des facteurs physiologiques telle que le vieillissement chronologique de la peau généralement liée à une perte de fonctionnalité des glandes sébacées et donc à une carence plus ou moins grande en sébum.

Cette sécheresse peut également avoir été acquise à l'issue d'un traitement thérapeutique par des composés tels que les dermocorticoïdes, les rétinoïdes ou des traitements de type puvathérapie et radiothérapie.

Enfin, cette sécheresse peut être constitutionnelle, c'est-à-dire manifestée chroniquement par le sujet ou être d'origine génétique à l'image de l'ichtyose, l'atopie, le psoriasis et l'hyperkératose.

Dans la mesure où les inventeurs ont également mis en évidence une action stimulatrice des glycérides à chaîne moyenne vis-à-vis de la sébogénèse, ces composés s'avèrent également avantageux pour traiter tous les désordres associés à une hyposéborrhée.

En conséquence, les glycérides à chaîne moyenne et les compositions selon l'invention peuvent être efficacement utilisés pour traiter des peaux manifestant une sécrétion et/ou excrétion insuffisante de sébum, de même que les désordres généralement associés à ce type de dérèglement comme par exemple un défaut de desquamation et/ou des manifestations micro-inflammatoires de type dermite.

Les compositions selon la description peuvent se présenter sous toutes les formes galéniques normalement utilisées pour le mode d'administration concerné.

Pour l'ingestion par voie orale, de nombreuses formes de réalisation de compositions orales et notamment de compositions médicamenteuses ou non et en particulier compléments alimentaires ou nutritionnels sont possibles. Leur formulation est réalisée par les procédés usuels pour produire des dragées, gélules, gels, émulsions, sèches ou liquides, comprimés, capsules ou solutions.

Les glycérides à chaîne(s) moyenne(s) peuvent aussi être incorporés dans des matrices alimentaires en vue de produire des aliments fonctionnels tels que des barres alimentaires, des aliments enrichis tels que des huiles, des beurres, des margarines, des poudres compactées, des fibres, des gommes, des pâtes à mâcher (chewing-gum), des fruits et légumes secs, des céréales, des chips, des viennoiseries, des biscuits, des crackers, ou encore sous la forme d'émulsion dans des boissons.

Dans le cas particulier où les compositions sont plus particulièrement destinées à une administration à des animaux, ces compositions peuvent se présenter sous la forme ' d'une composition alimentaire, de type support nutritionnel ou encore médicamenteuse. Quelque soit le type de composition, celle-ci sera formulée sous une forme galénique appropriée à son administration à la race animale concernée.

Les compositions peuvent être formulées avec les excipients et composants usuels pour de telles compositions orales, à savoir notamment des composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires sont connus dans ce domaine et ne font pas ici l'objet d'une description détaillée.

Dans les compositions, le composant glycéride à chaîne moyenne sous forme d'un unique composé ou d'un mélange, peut en outre être associé à une quantité efficace d'au moins un autre agent actif au niveau des matières kératiniques.

Par exemple, cet autre agent actif peut induire un effet indésirable comme l'apparition d'une peau sèche notamment par limitation de la production de sébum. A titre d'exemple de tels composés, on citera les corticoïdes, en particulier la cortisone, l'hydrocortisone et la bétaméthasone ; l'indométacine ; les dérivés de l'acide rétinoïque.

Comme actifs susceptibles d'être associés aux triglycérides à chaîne moyenne conformes à l'invention, on entend également les ingrédients communément utilisés et/ou autorisés dans les formes galéniques destinées à une administration par voie orale.

Ces ingrédients peuvent être choisis parmi les vitamines, les minéraux, les lipides essentiels et leurs dérivés, en particulier l'acide linoléique conjuguée (ou CLA), le caprenin (triglycéride d'acides caprique, caprylique et béhénique), les oligoéléments, les polyphénols, les flavonoïdes, les phyto-oestrogènes, les antioxydants tels que l'acide lipoïque et le coenzyme Q10, les caroténoïdes, les probiotiques, notamment les levures, les prébiotiques, les protéines et les acides aminés, les mono et polysaccharides, les amino-sucres, les phytostérols et alcools triterpéniques d'origine végétale.

Il s'agit, en particulier, des vitamines A, C, D, E, PP et du groupe B. Parmi les caroténoïdes, on choisit de préférence, le béta-carotène, le lycopène, la lutéine, la zéazanthine et l'astaxanthine. Les minéraux et oligo-éléments particulièrement mis en oeuvre sont le zinc, le calcium, le magnésium, le cuivre, le fer, l'iode, le manganèse, le sélénium, le chrome (III). Parmi les polyphénols, on retient aussi en particulier les polyphénols de raisin, de thé, d'olive, de cacao, de café, de pomme, de myrtille, de sureau, de fraise, de canneberge, et d'oignon. De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis. Les probiotiques sont choisis de préférence dans le groupe constitué par les lactobacilles et les bifidobactéries. Un probiotique convenant à l'invention peut également être choisi parmi les levures. Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stéaridonique, les acides gras oméga-3 de poisson à longue chaîne tels que l'EPA et le DHA, les acides gras conjugués issus de végétaux ou d'animaux tels que les CLA (Conjugated Linoleic Acid).

Selon un mode particulier de réalisation, ces ingrédients peuvent être choisis parmi l'acide linoléique conjuguée (CLA), les levures, le caprenin, les polyphénols, les flavanoïdes, les caroténoïdes autres que le beta-catotène, les phyto-oestrogènes, l'acide lipoïque et le coenzyme Q10, les probiotiques et notamment les levures, les amino-sucres, les phytostérols et alcools triterpéniques d'origine végétale. La présente invention concerne également un procédé ou méthode de traitement comprenant la mise en oeuvre de glycétride(s) à chaîne moyenne selon l'invention.

Un procédé de traitement non thérapeutique selon l'invention peut comprendre une administration unique.

Selon un autre mode de réalisation, l'administration est répétée, par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

On peut également envisager que ce procédé implique l'administration conjointe d'un autre produit destiné à avoir un effet bénéfique sur la matière kératinique considérée et notamment comprenant au moins un agent connu pour avoir une efficacité pour la prévention et/ou le traitement de la sécheresse des matières kératiniques. Il peut en particulier s'agir d'un actif tel que précisé précédemment. Cet autre produit peut être administré par voie orale ou par une voie distincte, par exemple topique.

De même, est décrit un procédé impliquant, conjointement à l'administration par voie orale de glycéride(s) à chaîne(s) moyenne(s), l'administration par voie topique ou parentérale de glycérides à chaîne(s) moyenne(s) identique(s) aux différents. Cette administration par voie topique peut être réalisée par l'application au niveau de la peau du sujet humain ou animal traité, de toute forme, galénique conventionnelle, telle que crème, lait, spray, gel corporel par exemple, contenant une quantité efficace en glycéride(s) à chaîne(s) moyenne(s).

Les exemples ci-après sont présentés à titre illustratif et non limitatif du domaine de l'invention.

### Exemple 1 : Différents modes de formulation par administration orale

Dans cet exemple, les TCM sont commercialisés par la Société COGNIS, sous la dénomination commerciale Delios^{®} V. Leurs caractéristiques physicochimiques principales sont les suivantes :

| **Composition physicochimique** | **Spécifications en poids** |
|---|---|
| Humidité | 0,1 % maximum |
| Répartition en acides gras | |
| C₆ : 0 | 2 % maximum |
| C₈ : 0 | 54-64% |
| C₁₀ : 0 | 34-46% |
| C₁₂ : 0 | 1% maximum |
| Indice d'acide (mg KOH/g) | 0,1 maximum |
| Indice de saponification (mg KOH/g) | 330-345 |
| Indice d'hydroxyle (mg KOH/g) | 5,0 maximum |
| Indice d'iode | 0,5 maximum |

### - Forme capsule molle

| Ingrédient/additif | Dosage (mg/capsule) |
|---|---|
| TCM | 65 |
| Huile d'olive | 25 |
| Vitamine E | 5 |

Il est possible de consommer 3 à 6 capsules par jour.

| Ingrédient/additif | Dosage (mg/capsule) |
|---|---|
| TCM | 45 |
| Huile de poisson | 30 |
| Huile de pépin de cassis | 10 |
| Vitamine E | 5 |

Il est possible de consommer 3 à 6 capsules par jour.

### - Gélule banderolée

| Ingrédient/additif | Dosage (mg/capsule) |
|---|---|
| TCM | 50 |
| Tristéarate | 70 |
| Succinate de vitamine E | 5 |
| Silice colloïdale Aerosil^{®} | 1 |

Il est possible de consommer 3 à 6 capsules par jour.

### - Gel unidose

| Ingrédient/additif | % en poids |
|---|---|
| TCM | 8 |
| Huile de bourrache | 4 |
| Sirop de sucre | 30 |
| Maltodextrine | 20 |
| Gomme xanthane | 0,7 |
| Lécithine de soja | 0,5 |
| Benzoate de sodium | 0,2 |
| Eau | QSP 100 |

Il est possible de consommer 100 à 400 ml par jour.

### Exemple 2 :

Une étude clinique est menée sur 80 femmes présentant une xérose hivernale, entre les mois de décembre et de mars, pour démontrer les effets bénéfiques d'une composition conforme à l'invention.

La composition testée correspond à des triglycérides à chaînes moyennes (TCM) conditionnés dans des capsules molles à l'image de celles proposées en exemple 1.

La dose quotidienne en TCM est équivalente à 2160 mg/jour et est administrée sous le forme de 4 capsules par jour, en deux prises matin et soir.

Les principaux critères d'inclusion des sujets de l'étude sont les suivants :
- Age : 50 à 80 ans (avec prise ou non d'un traitement hormonal de substitution),
- Score clinique de sécheresse cutanée : 1,5 à 3, et
- Mesure de l'hydratation au cornéomètre Mesure de l'hydratation au cornéomètre CMU125^{®} (appareil et méthode de Courage et Khazaka) : 25 et 45 u.a (unité arbitraire).

Le score clinique de sécheresse est réalisé selon un atlas cotant au niveau de la face externe des jambes l'intensité de la sécheresse avec la convention suivante, établie sur une échelle de 0 à 4,5.
- Score = 0/peau normale : relief cutané régulier. Aspect lisse.
- Score = 0,5/stade intermédiaire entre 0 et 1 : relief cutané légèrement irrégulier. Aspect faiblement rêche.
- Score = 1/peau déshydratée : relief cutané strié. Aspect peu rêche.
- Score = 1,5/stade intermédiaire entre 1 et 2 : aspect strié plus marqué et plus rêche.
- Score = 2/peau sèche : relief cutané strié et quelques squames. Aspect rêche.
- Score = 2,5/stade intermédiaire entre 2 et 3 : présence de squames sans écaille, très rêche.
- Score = 3/peau très sèche : squames nombreuses et quelques écailles. Aspect rugueux.
- Score = 3,5/stade intermédiaire entre 3 et 4 : présence d'écailles et de nombreuses squames. Aspect rugueux.
- Score = 4/peau extrêmement sèche : écailles très nombreuses. Aspect très rugueux.
- Score = 4,5/stade supérieur à 4 : pathologique.

Il a été demandé aux patients pendant toute la durée de l'étude, de ne pas modifier leurs habitudes alimentaires, de ne prendre aucun autre complément nutritionnel et de n'appliquer aucune composition cosmétique sur les zones étudiées.

La durée du traitement était de 12 semaines. Au cours de cette étude, il a été réalisé les investigations suivantes :
- investigations cliniques pour tous les sujets (40),
- investigations biochimiques et instrumentales pour la moitié de l'effectif (20 volontaires vivant en communauté présentant la même hygiène de vie pour garantir des critères d'inclusion homogènes sur l'ensemble de ces sujets, et
- renseignement d'un questionnaire d'auto-évaluation pour 40 sujets.

Une analyse statistique de cette étude a été réalisée sur chaque critère d'investigation en comparant l'évolution des paramètres entre J0 et J84.

Les investigations ont été respectivement réalisées selon les critères suivants :

### a) Investigations cliniques

- Examen clinique par un dermatologue. Cet examen évalue en particulier la sécheresse et la rugosité cutanées au niveau du visage, des avant-bras, des mains et des jambes (scorage en 4 points).

### b) Investigations instrumentales

- Cornéométrie au niveau de la jambe (critère d'inclusion),
- Sébumètre : mesure du sébum au niveau du front, et
- Etude de la mouillabilité au niveau de l'avant-bras.

La technique de mouillabilité utilisée a été décrite par P. Humbert et coll. (« A New Method To Measure In Vivo Human Skin Hydrophoby". Int. J. Cosmet. Sci., 2001 ; 23 :347-352 et "Skin Critical Surface Tension: A Way to Assess The Skin Wettability Qualitatively". Skin Res. Technol., 1996 ; 2 : 91-96).

### c) Investigations biochimiques

- Par prélèvements coton au niveau de la jambe, pour mesurer les lipides présents au niveau du stratum corneum, lipides du ciment intercoméocytaire et du sébum (squalène, triglycérides, glycérol, acides gras, cholestérol).

Les résultats obtenus à l'issue de ces investigations sont discutés ci-après.

### Résultats des investigations cliniques

Les résultats obtenus mettent en évidence, après 3 mois de prise d'une composition conforme à l'invention, une diminution très significative de la sécheresse cutanée en moyenne d'au moins 20 % sur les zones les plus exposées et donc les plus sensibles à cette problématique (jambes, visage, bras et avant-bras).

### Résultats des investigations instrumentales

Les résultats témoignent également d'une augmentation très significative du taux de sébum, d'environ de plus de 35 % après 3 mois de prise d'une composition conforme à l'invention.

Cette augmentation est à rapprocher des résultats positifs sur l'hydratation et la mouillabilité cutanée. En effet, l'augmentation de sébum participe à la restauration du film hydrolipidique et a une incidence positive sur l'hydratation. Enfin, la production de sébum se concrétise par une modification favorable de l'hydrophobicité de surface du stratum corneum.

### Résultats des investigations biochimiques

Les données acquises montrent également qu'après 3 mois de prise d'une composition conforme à l'invention, on observe une augmentation très significative du taux de squalène notamment d'au moins 75 %. Ce résultat est parfaitement en phase avec l'augmentation du taux du sébum mise en évidence par l'investigation instrumentale précédente.

On note également que l'administration orale de la composition selon l'invention favorise la synthèse des lipides du film hydrolipidique tels que les triglycérides, les acides gras, le glycérol et le cholestérol.

En conséquence, l'administration orale d'une composition conforme à l'invention révèle un effet positif sur la restauration du film hydrolipidique cutané avec une incidence très favorable sur la sécheresse cutanée.

Par ailleurs, les 40 volontaires interrogés sur les effets du produit relatent une amélioration significative du confort cutané entre J0 et J84 (amélioration de la douceur, sur les tiraillements et la souplesse de la peau).

Enfin, aucun évènement indésirable imputable à la composition n'a été mis en évidence traduisant une excellente tolérance du complément nutritionnel.

## Revendications

1. Utilisation non thérapeutique de glycéride(s) à chaîne moyenne, à titre d'actif, par voie orale, pour prévenir et/ou traiter les matières kératiniques sèches et/ou fragilisées, en particulier la peau.

2. Utilisation selon la revendication précédente, pour le traitement et/ou la prévention des désordres cutanés et/ou de l'unité pilosébacée liés à un défaut d'excrétion et/ou de sécrétion de sébum.

3. Utilisation selon la revendication 1, pour traiter les peaux sèches hypo-séborrhéiques.

4. Utilisation selon la revendication 1, pour prévenir et/ou traiter les démangeaisons et/ou tiraillements.

5. Utilisation selon la revendication 1, pour stimuler la sébogénèse.

6. Utilisation selon la revendication 1, pour prévenir et/ou traiter les fibres kératiniques sèches ou fragilisées.

7. Utilisation selon la revendication 1, pour prévenir et/ou réduire les rides liées à une sécheresse cutanée.

8. Utilisation de glycéride(s) à chaîne moyenne, en tant qu'actif, pour la préparation d'une composition thérapeutique pour l'administration orale, et destinée à prévenir et/ou traiter les peaux sèches et/ou fragilisées.

9. Utilisation selon la revendication précédente, pour prévenir et/ou traiter les démangeaisons et/ou tiraillements, pour prévenir et/ou traiter des désordres cutanés liés à un défaut d'excrétion et/ou de sécrétion de sébum, ou pour traiter les peaux sèches hyposéborrhéiques.

10. Utilisation selon l'une quelconque des revendications 1 à 9, **caractérisée en ce que** les glycérides à chaîne moyenne se présentent sous la forme d'un mélange de glycérides dont les acides gras possèdent de 6 à 12, et en particulier de 8 à 10 atomes de carbone.

11. Utilisation selon l'une quelconque des revendications précédentes, **caractérisée en ce que** les acides gras composant les glycérides possèdent des chaînes linéaires et saturées.

12. Utilisation selon l'une quelconque des revendications précédentes, **caractérisée en ce que** les glycérides à chaîne moyenne se présentent sous la forme d'un mélange comprenant de 40 à 90 %, et en particulier de 45 à 75 % et notamment de 50 à 70 % en poids d'acides gras en C₈.

13. Utilisation selon l'une quelconque des revendications précédentes, **caractérisée en ce que** les glycérides à chaîne moyenne se présentent sous la forme d'un mélange comprenant de 15 à 60 % en poids, et en particulier de 30 à 50 % en poids d'acides gras en C₁₀.

14. Utilisation selon l'une quelconque des revendications précédentes, **caractérisée en ce que** les glycérides se présentent sous la forme d'un mélange comprenant au maximum 2 % en poids d'acides gras en C₆.

15. Utilisation selon l'une quelconque des revendications précédentes, **caractérisée en ce que** lesdits glycérides se présentent sous la forme d'un mélange comprenant moins de 3 % et en particulier au plus 1 % d'acides gras en C₁₂.

16. Utilisation selon l'une quelconque des revendications précédentes, **caractérisée en ce que** les glycérides à chaîne moyenne sont choisis parmi les mono-glycérides, les diglycérides et les triglycérides à chaîne moyenne et leurs mélanges.

17. Utilisation selon l'une quelconque des revendications précédentes, **caractérisée en ce que** les glycérides à chaîne moyenne sont constitués ou comprennent au moins des triglycérides à chaîne moyenne.

18. Utilisation selon l'une quelconque des revendications précédentes, **caractérisée en ce que** les glycérides sont mis en oeuvre sous la forme d'une huile ou d'un mélange d'huiles végétale(s) les contenant.

19. Utilisation selon l'une quelconque des revendications précédentes, **caractérisée en ce que** ladite composition comprend de 0,1 à 100 % en poids, en particulier de 15 à 80 % en poids, notamment de 30 à 70 % en poids de glycéride(s) à chaîne moyenne par rapport au poids total de la composition.

20. Utilisation selon l'une quelconque des revendications précédentes, **caractérisée en ce que** la composition se présente sous la forme d'un complément alimentaire, d'une composition nutritionnelle ou sous la forme d'un aliment fonctionnel à visée de traitement des matières kératiniques destinés aux être humains ou aux animaux.

21. Utilisation selon l'une quelconque des revendications précédentes **caractérisée en ce qu'**elle comprend, à titre d'actif au moins un autre agent actif au niveau des matières kératiniques.

22. Utilisation selon la revendication précédente, **caractérisée en ce que** ledit agent actif est choisi parmi les vitamines, les minéraux, les lipides essentiels et leurs dérivés, en particulier l'acide linoléique conjuguée, le caprenin, les oligoéléments, les polyphénols, les flavonoïdes, les phyto-oestrogènes, les antioxydants tels que l'acide lipoïque et le coenzyme Q10, les caroténoïdes, les probiotiques, les prébiotiques, les , protéines et les acides aminés, les mono et polysaccharides, les amino-sucres, les phytostérols et alcools triterpéniques d'origine végétale.

23. Procédé de traitement non thérapeutique pour prévenir et/ou traiter les matières kératiniques sèches et/ou fragilisées, et en particulier les peaux sèches et/ou fragilisées, et/ou pour traiter les peaux sèches hypo-séborrhéiques, et/ou pour prévenir et/ou traiter les démangeaisons et/ou tiraillements et/ou pour stimuler la sébogénèse, et/ou pour le traitement et/ou la prévention des désordres cutanés et/ou de l'unité pilosébacée liés à un défaut d'excrétion et/ou de sécrétion de sébum, et/ou pour prévenir et/ou traiter les fibres kératiniques sèches ou fragilisées, et/ou pour prévenir et/ou réduire les rides liées à un sécheresse cutanée, comprenant l'administration par voie orale d'au moins une quantité efficace de glycéride(s) à chaîne moyenne.

24. Procédé selon la revendication 23, **caractérisé en ce qu'**il s'agit de triglycéride(s) à chaîne moyenne.

25. Procédé selon la revendication 23 ou 24, **caractérisé en ce que** les glycérides à chaîne moyenne sont administrés à des doses journalières pouvant aller de 0,5 mg à 100 g/j, en particulier de 1 mg à 10 g/j, et notamment de 5 à 2,5 g/j.

26. Procédé selon la revendication 23, 24 ou 25, **caractérisé en ce que** les triglycérides à chaîne moyenne sont administrés par voie orale.

27. Procédé selon l'une quelconque des revendications 23 à 26, **caractérisée en ce qu'**il comprend conjointement à ladite administration par voie orale l'administration par voie topique de glycérides à chaîne moyenne.

## Patentansprüche

1. Nicht-therapeutische orale Verwendung von mittelkettigen Glycerid(en) als Wirkstoff, zur Vorbeugung und/oder Behandlung trockener und/oder geschwächter keratinischer Substanzen, insbesondere der Haut.

2. Verwendung nach dem vorhergehenden Anspruch zur Behandlung und/oder Vorbeugung von Hautstörungen und/oder der Haartalgeinheit, die mit einem Sebum-Exkretions- und/oder -Sekretionsfehler verbunden sind

3. Verwendung nach Anspruch 1 zur Behandlung von trockenen hyposeborrhoischen Häuten.

4. Verwendung nach Anspruch 1 zur Vorbeugung und/oder Behandlung von Juckreiz und/oder Spannungsgefühlen.

5. Verwendung nach Anspruch 1 zur Stimulation der Sebogenese.

6. Verwendung nach Anspruch 1 zur Vorbeugung und/oder Behandlung trockener oder geschwächter keratinischer Fasern.

7. Verwendung nach Anspruch 1 zur Vorbeugung und/oder Reduktion von Falten auf Grund einer Hautaustrocknung.

8. Verwendung von mittelkettigen Glycerid(en) als Wirkstoff, zur Herstellung einer therapeutischen Zusammensetzung zur oralen Verabreichung und bestimmt zur Vorbeugung und/oder Behandlung von trockenen und/oder geschwächten Häuten.

9. Verwendung nach dem vorhergehenden Anspruch zur Vorbeugung und/oder Behandlung von Juckreiz und/oder Spannungsgefühlen zur Vorbeugung und/oder Behandlung von Hautstörungen und/oder der Haartalgeinheit, die mit einem Sebum-Exkretions- und/oder -Sekretionsfehler verbunden sind, zur Behandlung von trockenen hyposeborrhoischen Häuten.

10. Verwendung nach einem der Ansprüche 1 bis 9, **dadurch gekennzeichnet, dass** die mittelkettigen Glyceride in Form eines Glyceridgemisches vorliegen, dessen Fettsäuren von 6 bis 12, insbesondere von 8 bis 10 Kohlenstoffatome aufweisen.

11. Verwendung nach einem der vorhergehenden Ansprüche, **dadurch gekennzeichnet, dass** die Fettsäuren Glyceride aufbauen, die lineare und gesättigte Ketten besitzen.

12. Verwendung nach einem der vorhergehenden Ansprüche, **dadurch gekennzeichnet, dass** die mittelkettigen Glyceride in der Form eines Gemisches vorhanden sind, umfassend von 40 bis 90 Gew.-%, und insbesondere von 45 bis 75 Gew.-% und besonders von 50 bis 70 Gew.-% von C₈-Fettsäuren.

13. Verwendung nach einem der vorhergehenden Ansprüche, **dadurch gekennzeichnet, dass** die mittelkettigen Glyceride in der Form eines Gemisches vorhanden sind, umfassend von 15 bis 60 Gew.-%, und insbesondere von 30 bis 50 Gew.-% C₁₀-Fettsäuren.

14. Verwendung nach einem der vorhergehenden Ansprüche, **dadurch gekennzeichnet, dass** die Glyceride in der Form eines Gemisches vorhanden sind, umfassend maximal 2 Gew.-% C₆-Fettsäuren.

15. Verwendung nach einem der vorhergehenden Ansprüche, **dadurch gekennzeichnet, dass** die Glyceride in der Form eines Gemisches vorhanden sind, umfassend weniger als 3 Gew.-% und insbesondere höchstens 1 % C₁₂-Fettsäuren.

16. Verwendung nach einem der vorhergehenden Ansprüche, **dadurch gekennzeichnet, dass** die mittelkettigen Glyceride aus mittelkettigen Monoglyceriden, Diglyceriden, und Triglyceriden und ihren Gemischen ausgewählt sind.

17. Verwendung nach einem der vorhergehenden Ansprüche, **dadurch gekennzeichnet, dass** die mittelkettigen Glyceride aus mindestens mittelkettigen Triglyceriden aufgebaut sind oder sie umfassen.

18. Verwendung nach einem der vorhergehenden Ansprüche, **dadurch gekennzeichnet, dass** die Glyceride in der Form eines Öls oder eines Gemisches von Pflanzenölen, die sie enthalten, verwendet werden.

19. Verwendung nach einem der vorhergehenden Ansprüche, **dadurch gekennzeichnet, dass** die Zusammensetzung 0,1 bis 100 Gew.-%, insbesondere 15 bis 80 Gew.-%, besonders 30 bis 70 Gew.-% mittelkettige(s) Glycerid(e) bezogen auf das Gesamtgewicht der Zusammensetzung umfassen.

20. Verwendung nach einem der vorhergehenden Ansprüche, **dadurch gekennzeichnet, dass** die Zusammensetzung in Form eines Nahrungsergänzungsmittels, einer Nahrungszusammensetzung oder in Form eines funktionellen Lebensmittels zur Behandlung von keratinischen Substanzen für Menschen oder Tiere vorhanden ist.

21. Verwendung nach einem der vorhergehenden Ansprüche, **dadurch gekennzeichnet, dass** sie als Wirkstoff mindestens einen weiteren Wirkstoff der auf Keratinsubstanz-Niveau wirkt, umfasst.

22. Verwendung nach dem vorhergehenden Ansprüche, **dadurch gekennzeichnet, dass** der Wirkstoff ausgewählt ist aus Vitaminen, Mineralien, essentiellen Fetten und ihren Derivaten, insbesondere konjugierter Linolsäure, Caprenin, Oligoelementen, Polyphenolen, Flavonoiden, Phyto-Östrogenen, Antioxidantien, wie Liponsäure und Coenzym Q10, Carotinoiden, Probiotica, Präbiotika, Proteinen und Aminosäuren, Mono- und Polysacchariden, Amino-Zuckern, Phytosterolen und Triterpenalkoholen pflanzlicher Herkunft.

23. Verfahren zur nicht-therapeutischen Behandlung zur Vorbeugung und/oder Behandlung trockener und/oder geschwächter keratinischer Substanzen, insbesondere trockener und/oder geschwächter Haut, und/oder trockener hyposeborrhoischer Haut, und/oder zur Behandlung von Juckreiz und/oder Spannungsgefühlen, und/oder zur Stimulation der Sebogenese, und/oder Behandlung und/oder Vorbeugung von Hautstörungen und/oder der Haartalgeinheit, die mit einem Sebum-Exkretions- und/oder -Sekretionsfehler verbunden sind, und/oder zur Vorbeugung und/oder Behandlung von trockenen oder geschwächten keratinischen Fasern, und/oder zur Vorbeugung und/oder Reduktion von Falten auf Grund einer Hautaustrocknung, umfassend die orale Verabreichung von mindestens einer wirksamen Menge von mittelkettigem(n) Glycerid(en).

24. Verfahren nach Anspruch 23, **dadurch gekennzeichnet, dass** es sich um mittelkettige(s) Triglycerid(e) handelt.

25. Verfahren nach Anspruch 23 oder 24, **dadurch gekennzeichnet, dass** die mittelkettigen Glyceride in Tagesdosen verabreicht werden, die von 0,5 mg bis 100 g/Tag, insbesondere von 1 mg bis 10 g/Tag, und besonders von 5 bis 2,5 g/Tag reichen können.

26. Verfahren nach Anspruch 23, 24 oder 25, **dadurch gekennzeichnet, dass** die mittelkettigen Triglyceride oral verabreicht werden.

27. Verfahren nach einem der Ansprüche 23 bis 26, **dadurch gekennzeichnet, dass** es zusammen mit dem oralen Weg die Verabreichung von mittelkettigen Glyceriden auf topischem Weg umfasst.

## Claims

1. The non-therapeutic use of medium chain glycerides as active ingredient, via oral route, to prevent and/or treat dry and/or delicate keratinous substances, in particular the skin.

2. The use according to the preceding claim to treat and/or prevent cutaneous and/or pilo-sebaceous unit disorders related to defective excretion and/or secretion of sebum.

3. The use according to claim 1 to treat dry hypo-seborrheic skins.

4. The use according to claim 1 to prevent and/or treat itching and/or tightness.

5. The use according to claim 1 to stimulate sebogenesis.

6. The use according to claim 1 to prevent and/or treat dry and/or delicate keratinous fibers.

7. The use according to claim 1 to prevent and/or reduce wrinkles associated with skin dryness.

8. The use of medium chain glycerides as active ingredient to prepare a therapeutic composition for oral administration and intended to prevent and/or treat dry and/or delicate skins.

9. The use according to the preceding claim to prevent and/or treat itching and/or tightness, to prevent and/or treat skin disorders related to defective excretion and/or secretion of sebum, or to treat dry hypo-seborrheic skins.

10. The use according to any of claims 1 to 9, **characterized in that** the medium chain glycerides are in the form of a mixture of glycerides whose fatty acids have 6 to 12, in particular 8 to 10 carbon atoms.

11. The use according to one of the preceding claims, **characterized in that** the fatty acids composing the glycerides contain linear and saturated chains.

12. The use according to any of the preceding claims, **characterized in that** the medium chain glycerides are in the form of a mixture comprising 40 to 90 %, and in particular 45 to 75 % and more particularly 50 to 70 % by weight of C₈ fatty acids.

13. The use according to any of the preceding claims, **characterized in that** the medium chain glycerides are in the form of a mixture comprising 15 to 60 weight %, in particular 30 to 50 weight % of C₁₀ fatty acids.

14. The use according to any of the preceding claims, **characterized in that** the glycerides are in the form of a mixture comprising no more than 2 % by weight of C₆ fatty acids.

15. The use according to any of the preceding claims, **characterized in that** the said glycerides are in the form of a mixture comprising less than 3 % and in particular no more than 1 % of C₁₂ fatty acids.

16. The use according to any of the preceding claims, **characterized in that** the medium chain glycerides are chosen from medium chain monoglycerides, diglycerides and triglycerides and the mixtures thereof.

17. The use according to any of the preceding claims, **characterized in that** the medium chain glycerides are formed of or comprise at least medium chain triglycerides.

18. The use according to any of the preceding claims, **characterized in that** the glycerides are used in the form of a vegetable oil or mixture of vegetable oils in which they are contained.

19. The use according to any of the preceding claims, **characterized in that** the said composition comprises 0.1 to 100 % by weight, in particular 15 to 80 % by weight, more particularly 30 to 70 % by weight of medium chain glyceride(s) relative to the total weight of the composition.

20. The use according to any of the preceding claims, **characterized in that** the composition is in the form of a food supplement, a nutritional composition or in the form of a functional food for the treatment of keratinous substances intended for humans or animals.

21. The use according to any of the preceding claims, **characterized in that** it contains at least one other agent that is active on keratinous substances as active ingredient.

22. The use according to the preceding claim, **characterized in that** the said active agent is chosen from vitamins, minerals, essential lipids and the derivatives thereof, in particular conjugated linoleic acid, caprenin, trace elements, polyphenols, flavonoids, phytoestrogens, antioxidants such as lipoic acid and Q10 coenzyme, carotenoids, probiotics, prebiotics, proteins and amino acids, mono- and polysaccharides, amino-sugars, phytosterols and triterpene alcohols of vegetable origin.

23. A non-therapeutic treatment method to prevent and/or treat dry and/or delicate keratinous substances, in particular dry and/or delicate skins, and/or to treat dry hypo-seborrheic skins, and/or to prevent and/or treat itching and/or tightness, and/or to stimulate sebogenesis, and/or for the treatment and/or prevention of cutaneous and/or pilosebaceous unit disorders related to defective excretion and/or secretion of sebum, and/or to prevent and/or treat dry or delicate keratinous fibers, and/or to prevent and/or reduce wrinkles related to skin dryness, comprising the administration via oral route of at least an efficient quantity of medium chain glyceride(s).

24. The method according to claim 23, **characterized in that** it is about medium chain triglyceride(s).

25. The method according to claim 23 or 24, **characterized in that** the medium chain glycerides are administered in daily doses of up to 0.5 mg to 100 g/d, in particular 1 mg to 10 g/d, and more particularly 5 to 2.5 g/d.

26. The method according to claim 23, 24 or 25, **characterized in that** the medium chain triglycerides are administered via oral route.

27. The method according to any of claims 23 to 26, **characterized in that** it comprises the administration via topical route of medium chain glycerides jointly with the said administration via oral route.
